# EUROPÄISCHE PATENTSCHRIFT

(11) **EP 0 941 692 B1**
(45) Veröffentlichungstag und Bekanntmachung des Hinweises auf die Patenterteilung: **11.09.2002**
(21) Anmeldenummer: 99103575.9
(22) Anmeldetag: 24.02.1999
(51) Int. Cl.: A61B 3/12

(54) **Verfahren und Vorrichtung zur Untersuchung eines Augenabschnittes**
Method and device for examining the eye
Méthode et dispositif d'examen de l'oeil

(30) Priorität: 09.03.1998 DE 19810136; 20.03.1998 DE 19812297
(43) Veröffentlichungstag der Anmeldung: 15.09.1999
(73) Patentinhaber: Schwind eye-tech-solutions GmbH & Co. KG, 63801 Kleinostheim (DE)
(72) Erfinder: Birngruber, Reginald, 23564 Lübeck (DE); Scholz, Christian, 22846 Norderstedt (DE); Koch, Peter, 23552 Lübeck (DE); Engelhardt, Ralf, 23568 Lübeck (DE)
(74) Vertreter: Hofstetter, Alfons J., Dr.rer.nat.

(56) Entgegenhaltungen:
- EP-A- 0 659 383
- WO-A-87/05495
- US-A- 5 342 351

## Beschreibung

Die Erfindung betrifft ein Verfahren zur Untersuchung eines Augenabschnittes durch Belichtung desselben und Messung des rückgestreuten Lichtes mit Hilfe einer Punktlichtquelle, bei dem die Punktlichtquelle in einer Ebene abgebildet und zur Abtastung des zu untersuchenden Augenabschnittes abgelenkt und gerichtet wird und bei dem das am untersuchten Augenabschnitt reflektierte Licht erfaßt und durch Kohärenztomographie (OCT-Technik) ausgewertet wird. Ferner betrifft die Erfindung eine Vorrichtung zur Untersuchung eines Augenabschnittes mit einer Punktlichtquelle, einer optischen Abbildungseinrichtung zum Abbilden der Punktlichtquelle in einer Ebene und einer Abtasteinrichtung zur Erzeugung einer Abtastbewegung der Punktlichtquelle und einer optischen Kohärenztomographie-Einrichtung zur Auswertung des von dem zu untersuchenden Augenabschnitt reflektierten Lichtes.

Ein derartigers Verfahren und eine derartige Vorrichtung sind aus der US 5 493 109 bekannt.

Zur Untersuchung des Auges werden für den Vorderabschnitt und für den Hinterabschnitt unterschiedliche Aufnahmetechniken bisher verwendet. Für die Darstellung des Hinterabschnittes wird in der Ophthalmologie bislang eine Funduskamera oder eine Spaltlampe zusammen mit der Ophthalmoskopierlinse, z.B. einem Kontaktglas oder einer Volk-Linse verwendet. Die Darstellung des Vorderabschnittes ist mittels einer Spaltlampe möglich. Bekannt ist die Untersuchung des hinteren Augenabschnittes mit Hilfe einer Funduskamera auf der Basis der optischen Kohärenztomographie (OCT), wobei beispielsweiswe in der WO 92/19930 und den US-Patenten 5,537,162; 5,506,634; 5,493,109 und 5,321,501 entsprechende Apparaturen beschrieben sind. Die bekannten Apparaturen sind auf die Untersuchung des Hinterabschnittes (Fundus) des Auges optimiert.

Aus der JP 6-205 741 A ist ein ophthalmologisches Gerät bekannt, bei dem eine Spaltlampe als Beleuchtigungssystem und eine Meßeinrichtung vorgesehen sind. Die Meßeinrichtung beinhaltet eine Laserlichtquelle, einen Abtastspiegel, einen Strahlteiler und ein fotoelektrische Wandlerelement sowie einen Lichtermitter und ein Filter. Die Lichtquelle sendet einen Richtlaserstrahl für eine therapeutische Laserlichtquelle aus. Die Positionierung des Richtlaserstrahls wird von dem fotoelektrischen Wandlerelement und dem lichtemitierenden Element mit Hilfe eines Mikroskops überwacht. Wenn der Richtlaser eine gewünschte Position erreicht hat, wird der therapeutische Laser eingeschaltet und das Licht dieses Lasers über einen Spiegel in den Richtlaserstrahlgang eingekoppelt.

Um bei der OCT des hinteren Augenabschnittes Inhomogenitäten, insbesondere der brechenden Medien, zu vermeiden, ist ein komplizierter und langwieriger Einstellvorgang erforderlich, um den OCT-Strahlengang an den Inhomogenitäten des Auges vorbei auf den hinteren Augenabschnitt zu richten. Bei der Verwendung der OCT-Technik erfordert die Einstellung und der Zielvorgang, mit welchem der OCT-Strahlengang (Probenstrahl) auf den Augenhintergrund, insbesondere die Retina, gerichtet wird, eine spezielle Schulung und Einweisung sowie ständige Übung des untersuchenden Arztes.

Bei einer aus der Fig. 1 der US 5,537,162 bekannten Vorrichtung wird der Probenstrahlengang des OCT-Interferometers in der Schärfenebene (Bildebene) eines Spaltlampenmikroskops fokussiert. Mit Hilfe einer als Volk-Linse ausgebildeten Ophthalmoskopierlinse wird dann in Verbindung mit den brechenden Medien des Auges die Bildebene des Spaltlampenmikroskops auf die Retina des Auges abgebildet.

Die bekannte Vorrichtung kann nur zur OCT-Abtastung des hinteren Augenabschnittes verwendet werden. Dabei werden zwei getrennte Umlenkspiegel zur Erzeugung der Schnittbilder des Auges mit beliebiger Ausrichtung benützt. Hierbei ergibt sich die Schwierigkeit, daß die getrennten Umlenkspiegel einen Strahlversatz und bei nicht optimaler Beschichtung ein Doppelbild des Auges erzeugen. Außerdem wird der Arbeitsabstand zwischen dem Instrument und dem Auge des Patienten verkleinert, da sich beide Umlenkspiegel vor dem letzten optischen Bauelement der Spaltlampe befinden. Die Benutzung notwendiger optischer Einrichtungen, wie beispielsweise einer Ophthalmoskopierlinse, wird erschwert.

Aufgabe der Erfindung ist es daher, ein Verfahren und eine Vorrichtung der eingangs genannten Art zu schaffen, bei welchen der Einstell- und Zielvorgang für die Untersuchung des betreffenden Augenabschnittes mittels OCT-Technik erheblich vereinfacht wird.

Diese Aufgabe wird erfindungsgemäß beim Verfahren durch die kennzeichnenden Merkmale des Patentanspruches 1 und bei der Vorrichtung durch die kennzeichnenden Merkmale des Patentanspruches 3 gelöst. Die Unteransprüche beinhalten vorteilhafte Ausgestaltungen der Erfindung.

Die Erfindung beinhaltet die Kombination der herkömmlichen Untersuchung eines ausgewählten Augenabschnittes mittels der Spaltbeleuchtung (optisches Schnittbildverfahren) mit der OCT-Technik (kohärentes Schnittbildverfahren), die ein vorzugsweise durch kohärentes Licht erzeugtes Schnittbild vom untersuchten Augenabschnitt gewinnt. Hierbei können sowohl Vorderabschnitte (Hornhaut, Iris, Linse) als auch Hinterabschnitte (Glaskörper, Netzhaut, Aderhaut) des Auges mit dem gleichen Untersuchungsgerät mittels der OCT-Technik untersucht und erfaßt werden.

Die Erfindung verwendet in der Ophthalmologie etablierte Instrumente (Spaltlampe, Ophthalmoskopierlinse) für die Einstellung und den Zielvorgang des den Untersuchungsort am Augenabschnitt abtastenden OCT-Probenstrahles. Hierbei wird in vorteilhafter Weise ein Teil der Spaltlampenoptik für die Führung des OCT-Proben-Strahlenganges verwendet.

Da die Untersuchung mit der Spaltlampe für den Augenarzt eine Routineuntersuchung darstellt, wird der Einstell- und Zielvorgang, der bei der Anwendung herkömmlicher OCT-Untersuchungstechnik erheblich komplizierter und aufwendiger ist, wesentlich vereinfacht und im wesentlichen auf die bei der herkömmlichen Spaltlampentechnik erforderlichen Einstellvorgänge reduziert. Ferner ist es möglich, Augenuntersuchungen mit herkömmlicher Spaltlampentechnik und mit der OCT-Technik mit einem Untersuchungsgerät durchzuführen und dadurch eine bessere und genauere Korrelation zwischen Spaltlampenbildern und OCT-Schnittbildern herzustellen.

In vorteilhafter Weise können durch übliche Spaltlampenmanipulationen verschiedene Schnittbilder von einem Untersuchungsort am Auge nacheinander gewonnen werden. Diese Schnittbilder können parallel zueinander und nebeneinander liegend aufgenommen werden. Es ist jedoch auch möglich, sich überkreuzende Schnittbilder von einem Untersuchungsort am Auge zu gewinnen.

Bei der Erfindung wird eine punktförmige Beleuchtungs- und Meßeinrichtung (PBME), welche das Licht, insbesondere eines OCT-Interferometers einstrahlt und mißt und beispielsweise als Faserende eines Probenarmes des OCT-Interferometers ausgebildet sein kann, mittels einer Abbildungsoptik in den Strahlengang der Spaltbeleuchtung derartig eingekoppelt, daß die in herkömmlicher Weise zum Richten eines Spaltbildes einer Spaltlampe auf den zu untersuchenden Augenabschnitt verwendeten Optiken und Einstellmittel den Probenstrahl zusammen mit dem Spaltbild auf den zu untersuchenden Augenabschnitt richten. Bei der Messung des zu untersuchenden Augenabschnittes wird die Abtastbewegung durch die Ausrichtung des abgebildeten Spaltes vorgegeben. Insbesondere erfolgt die Abtastbewegung parallel bzw. entlang dem Spaltbild.

Hierzu können die Abbildung der PBME und die Abbildung des Spaltes mit Hilfe einer Einkoppeloptik bzw. Überlagerungsoptik, die beispielsweise als dichroitischer Teiler (Spiegel) ausgebildet sein kann, überlagert werden. Die PBME und der Spalt können entweder in einer gemeinsamen Ebene oder in zueinander konjugierten Ebenen abgebildet werden, um anschließend mittels der Spaltabbildungsoptik gemeinsam auf den zu untersuchenden Augenabschnitt gerichtet zu werden.

Die Spaltlampe kann gegenüber der Einkoppeloptik bzw. Überlagerungsoptik in der Weise angeordnet sein, daß die gemeinsame Ebene, in welcher der Spalt zusammen mit der PBME abgebildet wird, vor der Überlagerungsoptik in dem von der punktförmigen Emissionsfläche der PBME kommenden OCT-Strahlengang liegt. Zur Abtastung reicht es aus, daß der OCT-Probenstrahl in der gemeinsamen Ebene (konjugierten Spaltebene) in Richtung des abgebildeten Spaltes abgelenkt wird. Bei einer weiteren Ausführungsform kann die Abtastung in der Weise erfolgen, daß die abgebildete PBME in einer beliebigen, relativ zum abgebildeten Spalt jedoch fest definierten Kurvenform bewegt wird. Dies kann mit Hilfe beweglicher Schwenkspiegel geschehen. Der Überlagerungsoptik nachgeordnet erfolgt die Führung des OCT-Probenstrahls gemeinsam mit den der Spaltbeleuchtung zugeordneten optischen Baugruppen, nämlich der Spaltabbildungsoptik. Mit Hilfe des zur Spaltlampe gehörenden optischen Systems erfolgt die Ausrichtung des abtastenden Belichtungsstrahls im vorderen Augenabschnitt (Hornhaut, Iris, Linse). Für Untersuchungen am hinteren Augenabschnitt erfolgt die Abbildung des von der Spaltabbildungsoptik von der PBME und dem Spalt erzeugten Bildes mittels einer Ophthalmoskopierlinse, z.B. einer Volk-Linse und den brechenden Medien des Auges (Fig. 1 der US 5,537,162).

Die im OCT-Probenstrahiengang vor der Einkoppeloptik bzw. Überlagerungsoptik liegende Abbildungs- und Führungsoptik kann in der Weise mit der Spaltlampe, insbesondere dem den Spalt- und die Spaltbeleuchtung aufweisenden Spaltlampenkopf, verbunden sein, daß bei der Bewegung (Verdrehen, Schwenken, Kippen) des Spaltlampenkopfes und der daraus resultierenden Bewegung des Spaltes, z.B. Verdrehen um die optische Achse, gleichzeitig auch die Abbildungs- und Führungsoptik, insbesondere die zum Abtasten längs des Spaltes verwendeten Abtastmittel, mitgedreht bzw. mitbewegt werden. Hierdurch wird erreicht, daß der OCT-Probenstrahl bei jeder Spaltstellung in der gemeinsamen Ebene abgebildet wird. In gleicher Weise kann auch die Spaltabbildungsoptik mitbewegt werden. In vorteilhafter Weise kann sich die ausschließlich für die OCT erforderliche Optik, z.B. das OCT-Interferometer, entfernt vom Untersuchungsplatz befinden, so daß es zu keiner Behinderung am Untersuchungsplatz während der Untersuchung am Patienten kommt.

Bei der Verwendung einer Volk-Linse mit der Spaltlampe hängt die Fokuslänge der Abbildung im wesentlichen vom Abstand der Volk-Linse zur Spaltlampe ab. Bei korrekter Abstimmung dieses Abstandes bleibt die Abbildung auch bei verändertem Abstand zwischen Meßaufbau und Patient erhalten, da zwischen der Volk-Linse und dem rechtsichtigen Patientenauge der Strahlengang parallel ist. Dieser Abstand soll bei dem OCT-Meßverfahren, welches entfernungssensitiv ist, variabel sein. Das Ziel einer weiteren Ausgestaltung der Erfindung besteht darin, bei gefundener Einstellung der Fokuslage diesen Abstand und damit den optischen Weg im Probenstrahlengang ohne Änderung der Fokuslage zu variieren.

Dieses Ziel wird bei einer Ausgestaltung der Erfindung dadurch erreicht, daß zur Abbildung eines den Spalt und die PBME enthaltenden Zwischenbildes auf den Fundus des Auges, z.B. die Retina des Auges, die Volk-Linse in einem bestimmten Abstand zu diesem Zwischenbild fixierbar ist. Dies wird durch eine Verbindung der Volk-Linse mit der Spaltlampe, insbesondere dem Spaltlampenkopf, herbeigeführt, mit welcher der Abstand der Volk-Linse vom Zwischenbild für die jeweilige Untersuchung am Auge fest eingestellt werden kann. Hierzu dient eine Haltevorrichtung, in welcher die Volk-Linse unter Beibehaltung eine bestimmten Abstandes zum Spalt, in allen Freiheitsgraden beweglich gehalten ist. In vorteilhafter Weise wird hierzu eine kardanische Aufhängung der Volk-Linse in der Haltevorrichtung benutzt.

Die Haltevorrichtung kann aus einer vertikalen und einer horizontalen Stange, die gelenkig miteinander verbunden sind, gebildet sein.

Anhand der Figuren wird die Erfindung noch näher erläutert. Es zeigt:
- Fig. 1:: schematisch ein Ausführungsbeispiel der Erfindung; und
- Fig. 2:: eine Ausführungsform für eine Ausgestaltung der Erfindung.

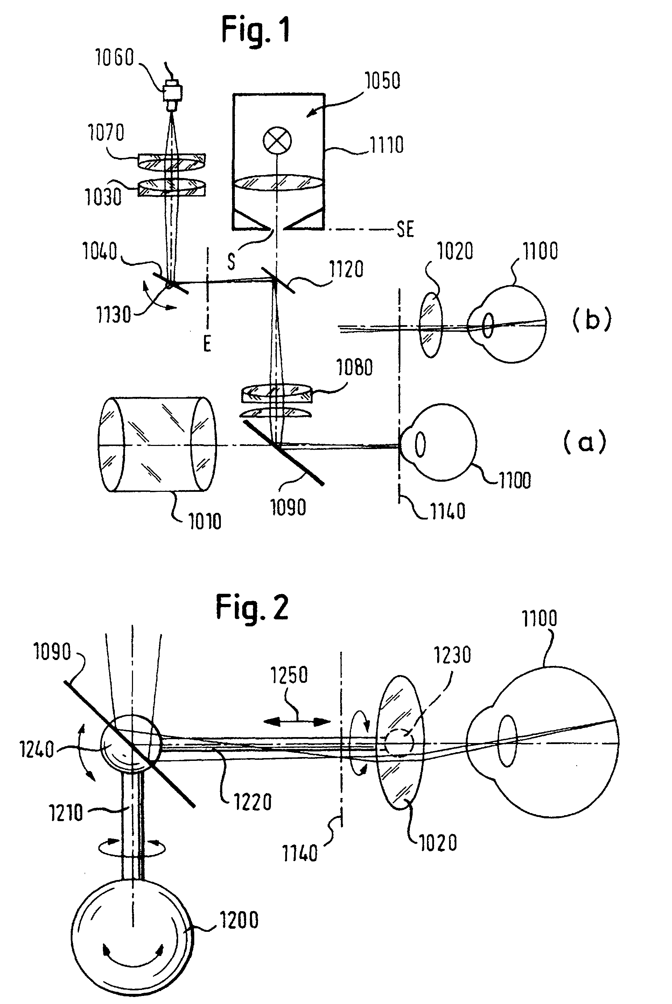

Das in der Fig. 1 schematisch dargestellte Ausführungsbeispiel zeigt eine Vorrichtung zur Untersuchung von Abschnitten, insbesondere Vorderabschnitt und Hinterabschnitt eines Auges 1100. Hierzu wird von einer punktförmigen Beleuchungs-Meßeinrichtung (PBME) 1060 kohärentes Licht, insbesondere ein Laserstrahl, ausgesendet. Die PBME 1060 kann als Faserende einer Licht leitenden Faser eines optischen Kohärenztomographie-Interferometers (OCT-Interferometers) ausgebildet sein. Das aus dem Faserende bzw. der PBME 1060 austretende Licht wird mit einer Kollimatoroptik 1070 kollimiert und anschließend mit einer Fokussierungsoptik 1030 in einer Ebene E fokussiert bzw. mit einer Abbildungsoptik in der Ebene E abgebildet. Zwischen der Ebene E und dem aus Fokussierungsoptik 1030 und Kollimatoroptik 1070 bestehendes Abbildungssystem befindet sich eine Abtasteinrichtung 1040, welche beim dargestellten Ausführungsbeispiel als schwenkbarer Spiegel ausgebildet ist. Zur Erzeugung der Abtastbewegung kann der Spiegel von beispielsweise einem Galvanometer angetrieben sein und in einem Schwenklager 1130 gelagert sein.

Anschließend wird der Laserstrahl mit einer Einkoppeloptik bzw. Überlagerungsoptik 1120, welche als dichroitischer Spiegel ausgebildet sein kann, mit dem BeleuchtungsStrahlengang einer Spaltlampe überlagert.

Zur Spaltlampe gehört ein Spaltlampenkopf 1110, der in einer Spaltebene SE einen Spalt S aufweist. Der Spalt S wird von einem Beleuchtungssystem 1050 beleuchtet. Mit de Überlagerungsoptik 1120 (dichroitischer Spiegel) wird die Ebene E in eine zur Spaltebene SE konjugierte Ebene gebracht.
Auf diese Weise wird die PBME 1060, welche das Faserende eines Probenarmes des OCT-Interferometers sein kann, mit dem Spalt S bzw. der Spaltebene SE der Spaltlampe zusammengeführt.

Eine der Überlagerungsoptik 1120 nachfolgende Spaltabbildungsoptik 1080, welche zur Spaltbeleuchtungsoptik gehört, bildet über eine Umlenkoptik 1090, welche als Umlenkspiegel ausgebildet sein kann, die konjugierten Ebenen E und SE in den zu untersuchenden Augenabschnitt des Auges 1100 in einer Bildebene 1140 ab. In der unteren Darstellung (a) der Fig. 1 befindet sich die Ebene 1140 im vorderen Augenabschnitt.

Für Untersuchungen am hinteren Augenabschnitt (z.B. Retina) erfolgt, wie die Darstellung (b) in Fig. 1 zeigt, eine weitere Abbildung der Bildebene 1140 mittels einer beispielsweise als Volk-Linse 1020 ausgebildeten Ophthalmoskopierlinse, die zwischen dem Patientenauge 1100 und der Bildebene 1140 angeordnet ist, und mittels den brechenden Medien des Auges auf den hinteren Augenabschnitt.

Der Spaltlampenkopf 1110 und das Schwenklager 1130 für die Abtasteinrichtung 1040 sowie die Abbildungsoptik (Fokussierungslinsensystem 1030, Kollimatorlinsensystem 1070) für die PBME 1060 können starr miteinander verbunden sein. Ferner können die Spaltabbildungsoptik 1080 und die Überlagerungsoptik 1120 starr mit dem Spaltlampenkopf 1110 verbunden sein. Bei einer Bewegung (Verdrehen, Kippen, Schwenken) des Spaltlampenkopfes 1110 und der daraus resultierenden Bewegung des Spaltes S wird damit gleichzeitig die Abtasteinrichtung 1040 mitbewegt. Ferner werden die zugeordneten Optiken 1030, 1070 und 1080 mitbewegt, so daß für jede beliebige Spaltstellung die Zusammenführung der PBME 1060 mit dem Spalt S der Spaltlampe durch die Überlagerungsoptik 1120 gewährleistet wird. Auf diese Weise erreicht man allein durch Betätigen der Spaltlampe eine auf den Untersuchungsort am Auge gerichtete Ausrichtung des OCT-Strahlenganges für die Abtastung des untersuchenden Augenabschnitts. Bei der Abtastung wird der von der PBME kommende Lichtstrahl durch die Spaltabbildungsoptik 1080 und die Umlenkeinrichtung 1090 zum Untersuchungsort am Augenabschnitt geführt. Die Abtastung des Untersuchungsortes erfolgt entlang dem abgebildeten Spalt, wobei die Abtastbewegung durch die beispielsweise als Schwenkspiegel ausgebildete Abtasteinrichtung 1040 erfolgt. Der reflektierte Meßstrahl wird über die oben erläuterten optischen Baugruppen zur PBME zurückgeführt, und durch Auswertung des Meßstrahles wird ein Schnittbild vom Untersuchungs- bzw. Meßort erzeugt. Hierdurch erreicht man durch Kombination von Spaltlampentechnik und Abtasttechnik die Herstellung eines optischen Schnittbildes. Es können durch Mehrfachabtastung nacheinander mehrere Schnittbilder, die beispielsweise parallel nebeneinanderliegen oder auch gekreuzt zueinanderliegen, hergestellt werden.

Bei einer weiteren Ausführungsform können z.B. durch Integration mindestens eines zusätzlichen Schwenkspiegels beliebige Muster mit einer definierten Lage relativ zum Spaltbild abgetastet werden.

In der Fig. 2 ist ein Ausführungsbeispiel für eine Haltevorrichtung schematisch dargestellt, mit welcher die Volk-Linse 1020 gegenüber der Bildebene 1140 der Spaltlampe in einem festen Abstand, der für die jeweilige Untersuchung einstellbar ist, gehalten werden kann. Die Haltevorrichtung wird gebildet von einer horizontalen Stange 1220, an welcher, insbesondere am Ende, die Volk-Linse 1020 in einem Kardangelenk 1230 in allen Freiheitsgraden um einen Drehpunkt beweglich gelagert ist. Hierdurch wird die Bewegung der Volk-Linse in jede beliebige Lage für eine optimale Justage des Belichtungsvorgangs mit dem abtastenden Laserstrahl der OCT-Meßeinrichtung ermöglicht. Die horizontale Stange 1220 ist über ein Gelenk 1240, insbesondere in Form eines Scharniers, mit einer vertikalen Stange 1210 verbunden. Das Gelenk 1240 befindet sich im Schnittpunkt der Umlenkoptik 1090. Mit Hilfe des Gelenkes 1240 ist die Zentrierung der Volk-Linse 1020 stufenlos einstellbar. Die vertikale Stange 1210 ist mittels eines Halters 1200, der als magnetischer Halter ausgebildet sein kann, drehbar an der Spaltlampe fixiert. Hierdurch wird die Beibehaltung eines eingestellten Abstandes der Volk-Linse 1020 gegenüber der Bildebene 1140 der Spaltlampe gewährleistet.

Der axiale Abstand der Volk-Linse 1020 gegenüber der Bildebene 1040 läßt sich durch Drehen des gesamten Halters 1200 grob voreinstellen. Eine Feineinstellung des axialen Abstandes sowie eine Anpassung der Fokuslage, beispielsweise bei Fehlsichtigkeit des Patienten, kann durch eine Längenverstellung (Doppelpfeil 1250) der horizontalen Stange 1220 gewährleistet werden.

Hierdurch ist es möglich, bei gefundener Einstellung der Fokuslage den optischen Weg ohne Veränderung der Fokuslage zwischen der Volk-Linse 1020 und dem zu untersuchenden Abschnitt am Patientenauge 1100 zu variieren.

Die beschriebene Haltevorrichtung für die Volk-Linse erlaubt es dem Arzt, während der Untersuchung andere Vorgänge zu beobachten bzw. zu steuern. Durch die magnetische Adaption der Haltevorrichtung ist ein schneller und problemloser Wechsel zwischen rechten und linken Augen möglich, da der Magnethalter je nach Bedarf an einer der beiden Seiten der Spaltlampe angebracht werden kann. Durch die feste Verbindung der Volk-Linse 1020 mit der Spaltlampe, insbesondere dem Spaltlampenkopf 1110, bleibt der Abstand zwischen der Volk-Linse 1020 und der Bildebene 1040 bei der Bewegung der Spaltlampe unverändert.

## Patentansprüche

1. Verfahren zur Untersuchung eines Augenabschnittes durch Belichtung desselben und Messung des rückgestreuten Lichtes mit Hilfe einer Punktlichtquelle, bei dem die Punktlichtquelle in einer Ebene abgebildet und zur Abtastung des zu untersuchenden Augenabschnittes abgelenkt und gerichtet wird und bei dem das am untersuchten Augenabschnitt reflektierte Licht erfaßt und durch Kohärenztomographie (OCT-Technik) ausgewertet wird,
**dadurch gekennzeichnet ,**
**daß** die Punktlichtquelle zusammen mit dem Spalt einer Spaltlampe in konjugierten Ebenen abgebildet und anschließend zu einer gemeinsamen Abbildung überlagert werden oder gemeinsam in einer Ebene abgebildet werden, daß die gemeinsame Abbildung von Spalt und Punktlichtquelle mittels einer Spaltabbildungsoptik auf den zu untersuchenden Augenabschnitt gerichtet und bei der Abtastung geführt wird und daß das reflektierte Licht über denselben Strahlengang zur Punktlichtquelle für die Kohärenztomographie zurückgeführt wird.

2. Verfahren nach Anspruch 1, **dadurch gekennzeichnet, daß** am zu untersuchenden Augenabschnitt mehrere unterschiedliche Abtastvorgänge nacheinander durchgeführt werden.

3. Vorrichtung zur Untersuchung eines Augenabschnittes mit einer Punktlichtquelle, einer optischen Abbildungseinrichtung zum Abbilden der Punktlichtquelle in einer Ebene und einer Abtasteinrichtung zur Erzeugung einer Abtastbewegung der Punktlichtquelle und einer optischen Kohärenztomographie-Einrichtung zur Auswertung des von dem zu untersuchenden Augenabschnitt reflektierten Lichtes,
**dadurch gekennzeichnet ,**
**daß** eine Einkoppeloptik (1120), welche die Abbildung (E) der Punktlichtquelle (1060) und einen Spalt (S) einer Spaltlampe über eine Spaltabbildungsoptik (1080) in eine gemeinsame Bildebene (1140) zusammenführt und gemeinsam auf den zu untersuchenden Augenabschnitt abbildet, vorgesehen ist, daß die Abtasteinrichtung (1040) in der Weise ausgebildet ist, daß die abgebildete Punktlichtquelle beim Abtastvorgang in Längsrichtung des abgebildeten Spaltes oder in einer relativ zum abgebildeten Spalt vorgegebenen Richtung bewegt wird, und daß das reflektierte Licht über denselben Strahlengang zur Punktlichtquelle (1060) zurückgeführt ist.

4. Vorrichtung nach Anspruch 3, **dadurch gekennzeichnet, daß** die Spaltabbildungsoptik (1080) in Richtung des auf das zu untersuchende Auge nachfolgend zur Überlagerungsoptik (1120) angeordnet ist.

5. Vorrichtung nach Anspruch 3 oder 4, **dadurch gekennzeichnet, daß** die gemeinsame Bildebene (E), in welcher die Spaltebene (SE) und die Punktlichtquelle (1060) zusammengeführt sind, in Richtung des auf das zu untersuchende Auge vor der Überlagerungsoptik (1120) liegt.

6. Vorrichtung nach einem der Ansprüche 3 bis 5, **dadurch gekennzeichnet, daß** mit der Bewegung des Spaltes (S) und der dazugehörigen Spaltabbildungsoptik (1080) eine Bewegung der die Punktlichtquelle (1060) in die gemeinsame Bildebene (E) abbildenden Abbildungs- und Führungseinrichtungen (1030, 1040, 1070, 1130) gekoppelt ist.

7. Vorrichtung nach einem der Ansprüche 3 bis 5, mit einer Detektoreinrichtung zur Erfassung des von dem zu untersuchenden Augenabschnitt reflektierten Lichts,
**dadurch gekennzeichnet ,**
**daß** zur Abbildung eines den Spalt (S) und die Punktlichtquelle (1060) enthaltenden Zwischenbildes (1140) auf den Fundus des Auges eine Volk-Linse (1020) zwischen dem zu untersuchenden Auge und der Zwischenbildebene (1140) in einem bestimmten Abstand zur Zwischenbildebene (1140) angeordnet ist.

8. Vorrichtung nach Anspruch 7, **dadurch gekennzeichnet, daß** die Volk-Linse (1020) über eine Haltevorrichtung (1210, 1220) in einem bestimmten Abstand zur Zwischenbildebene (1140) befestigbar ist.

9. Vorrichtung nach Anspruch 7 oder 8, **dadurch gekennzeichnet, daß** die Volk-Linse (1020) in einer kardanischen Aufhängung (1030) in der Haltevorrichtung (1210, 1220) gelagert ist.

## Claims

1. Method for examining an eye section by exposing the section to light with a point light source and measuring the backscattered light, the point light source being imaged in a plane, deflected, and directed for scanning the eye section to be examined, the light reflected on the eye section to be examined being sensed and being evaluated by coherence tomography (OCT technology),
**characterized in that**
the point light source is imaged together with a slit of a slit lamp in conjugated planes, which are subsequently superimposed into a joint image or jointly imaged in a plane, the joint imaging of the slit and the point light source is directed by way of a slit imaging lens system to the eye section to be examined which is guided during scanning and the reflected light is returned by way of the same beam path to the point light source for the coherence tomography.

2. Method according to claim 1, **characterized in that** several different scanning operations are carried out successively on the eye section to be examined.

3. Device for examining an eye section with a point light source, an optical imaging device for imaging the point light source in a plane and a scanning device for generating a scanning movement of the point light source and an optical coherence tomography device for evaluating the light reflected by the eye section to be examined,
**characterized in that**
a coupling-in lens (1120) is provided which combines the imaging (E) of the point light source (1060) and a slit (S) of a slit lamp by way of a slit imaging lens system (1080) into a joint image plane (1140) and images them jointly on the eye section to be examined, the scanning device (1040) is constructed such that the imaged point light source is moved during a scanning operation in a longitudinal direction of the imaged slit or in a direction defined relative to the imaged slit, and the reflected light is returned by the same beam path to the point light source (1060).

4. Device according to claim 3, **characterized in that** the slit imaging lens system (1080) is arranged, in the direction of the eye to be examined, following the superimposing lens system (1120).

5. Device according to claim 3 or 4, **characterized in that** the joint image plane (E), in which the slit plane (SE) and the point light source (1060) are combined, is situated, in the direction of the eye to be examined, in front of the superimposing lens system (1120).

6. Device according to one of the claims 3 to 5, **characterized in that** movement of imaging and guiding devices (1030, 1040, 1070, 1130) imaging the point light source (1060) into the joint image plane (E) is coupled with movement of the slit (S) and the associated slit imaging lens system (1080).

7. Device according to one of the claims 3 to 5 with a detector device for detecting the light reflected by the eye section to be examined,
**characterized in that**
a Volk lens (1020) is arranged between the eye to be examined and the intermediate image plane (1140) containing the slit (S) and the point light source (1060), which images the intermediate image (1140) onto the fundus of the eye, the Volk lens being arranged at a defined distance with respect to the intermediate image plane (1140).

8. Device according to claim 7, **characterized in that** the Volk lens (1020) can be fastened by a holding arrangement (1210, 1220) at a defined distance from the intermediate image plane (1140).

9. Device according to claim 7 or 8, **characterized in that** the Volk lens (1020) is disposed in a cardanic mounting (1030) in the holding arrangement (1210, 1220).

## Revendications

1. Procédé pour l'examen d'une section d'oeil par son illumination et par mesure de la lumière réfléchie, à l'aide d'une source de lumière ponctuelle, dans lequel la source de lumière ponctuelle est reproduite dans un niveau et est déviée et dirigée pour l'exploration de la section d'oeil à examiner, et dans lequel la lumière réfléchie par la section d'oeil examinée est capturée et exploitée par tomographie optique cohérente (OCT),
**caractérisé en ce que**
la source de lumière ponctuelle est reproduite conjointement avec la fente d'une lampe à fente dans des niveaux conjugués puis sont superposées en une image commune ou reproduites ensemble en un niveau, que l'image commune de la fente et de la source de lumière ponctuelle au moyen d'une optique de reproduction d'image de fente est dirigée vers la section d'oeil à examiner et est conduite par l'exploration et que la lumière réfléchie est reconduite par le même chemin de rayons vers la source de lumière ponctuelle pour la tomographie cohérente.

2. Procédé selon la revendication 1 **caractérisé en ce que** plusieurs processus d'exploration différents sont effectués successivement sur la section d'oeil à examiner.

3. Dispositif pour l'examen d'une section d'oeil avec une source de lumière ponctuelle d'une installation de reproduction d'image optique pour reproduire la source de lumière ponctuelle dans un niveau et une installation d'exploration pour créer un mouvement d'exploration de la source de lumière ponctuelle et une installation de tomographie cohérente pour l'exploitation de la lumière réfléchie par la section d'oeil à examiner,
**caractérisé en ce que**
une optique d'accouplage (1120), qui réunit en un niveau d'image commun (1140) l'image (E) de la source de lumière ponctuelle(1060) et une fente (S) d'une lampe à fente au travers d'une optique de reproduction d'image à fente (1080), et les reproduit ensemble sur la section d'oeil à examiner, est prévue, que l'installation d'exploration (1040) est formée de manière telle que la source de lumière ponctuelle reproduite lors du processus d'exploration est déplacée dans le sens de la longueur de la fente reproduite ou selon une direction prédéterminée relativement à la fente reproduite, et que la lumière réfléchie est reconduite par le même chemin de rayons vers la source de lumière ponctuelle (1060).

4. Dispositif selon la revendication 3, **caractérisé en ce que** l'optique de reproduction d'image de fente (1080) est arrangée en direction de l'oeil à examiner en aval de l'optique de superposition (1120).

5. Dispositif selon la revendication 3 ou 4, **caractérisé en ce que** le niveau d'image commun (E), dans lequel le niveau de fente (SE), et la source de lumière ponctuelle (1060) sont réunis, est situé en direction de l'oeil à examiner en amont de l'optique de superposition (1120).

6. Dispositif selon l'une des revendications 3 à 5, **caractérisé en ce que**, avec le mouvement de la fente (S) et de l'optique de reproduction d'image en fente (1080) correspondante, un mouvement des moyens de reproduction et de guidage (1030, 1040, 1070, 1130) reproduisant la source de lumière ponctuelle (1060) dans le niveau d'image commun (E) est accouplé.

7. Dispositif selon l'une des revendications 3 à 5comprenant un moyen de détection pour acquérir la lumière réfléchie par la section d'oeil à examiner
**caractérisé en ce que**
pour la reproduction sur le fond de l'oeil d'une image intermédiaire (1140) contenant la fente (S) et la source de lumière ponctuelle (1140), une lentille de Volk (1020) est disposée entre l'oeil à examiner et le niveau d'image intermédiaire (1140) à une certaine distance du niveau d'image intermédiaire (1140).

8. Dispositif selon la revendication 7 **caractérisé en ce que** la lentille de Volk (1020) peut se fixer à une certaine distance du niveau d'image intermédiaire (1140) au moyen d'un dispositif de maintien (1210, 1220).

9. Dispositif selon la revendication 7 ou 8 **caractérisé en ce que** la lentille de Volk (1020) est logée dans une suspension à cardan (1030) dans le dispositif de maintien (1210, 1220).
